# EUROPEAN PATENT SPECIFICATION

(11) **EP 0 440 047 B1**
(45) Date of publication and mention of the grant of the patent: **25.05.1994**
(21) Application number: 91100541.1
(22) Date of filing: 18.01.1991
(51) Int. Cl.: A61M 5/50, A61M 5/32, A61B 19/02

(54) **Medical appliance with seal portion**
Medizinische Einrichtung mit Versiegelungsabschnitt
Dispositif médical avec partie de scellement

(30) Priority: 24.01.1990 JP 5239/90 U; 26.11.1990 JP 123853/90 U
(43) Date of publication of application: 07.08.1991
(73) Proprietor: NISSHO CORPORATION, Osaka-shi (JP)
(72) Inventor: Adachi, Shinichi, Hinai-machi, Kitaakita-gun, Akita-ken (JP); Hagiwara, Kaoru, Odate-shi, Akita-ken (JP); Hosono, Hisashi, Ashikaga-shi, Tochigi-ken (JP)
(74) Representative: Türk, Gille, Hrabal, Leifert

(56) References cited:
- US-A- 3 149 717
- US-A- 3 272 322

## Description

The present invention relates to a medical appliance or device with a seal portion, and more particularly to a medical appliance with a seal portion having a protector to maintain sterility of a appliance such as an injection needle, a liquid-introducing needle and a connector and being capable of ascertaining that the protector has not been opened in order to prove sterility of the appliance.

Medical appliances such as injection needles, liquid-introducing needles and connectors which directly contact with tissue, blood, liquid drug, and the like are sterilized. A cap, i.e. protector, for protecting a needle or a connecting end is put on the above-mentioned appliance in order to maintain sterility thereof. The protector is usually attachable to and detachable from the needle or connecting end. That is to say, the protector can be put on the appliance again after it is once detached from the appliance. Accordingly, sterility of the medical appliance is assured as long as it is contained in a bag such as blister pack. However, once the medical appliance is taken out from the bag, sterility thereof cannot be assured even if the protector is put on the medical appliance.

For keeping a hypodermic needle sterile, a container is known (US-A-3 149 717) which comprises a shell for receiving the hypodermic needle and a cap for closing the open end of the shell. The cap is locked to the shell by detents inserted through slots provided in a flange at the upper end of the shell. Such detents can be broken off the cap upon twisting the cap relative to the flange in order to open the entire container. However, as the detents are not readily recognizeable, in particular if the container is viewed more or less from above, it is not easily apparent for a user whether the container had already been opened or not prior to use of the neddle stored therein.

The present invention was made to solve the above-mentioned problem, and it is the object of the invention to provide a medical appliance having such a structure that can easily ascertain, when using the medical appliance, that the protector has not yet been put off and can accordingly assure maintenance of sterility in a portion to be protected.

In accordance with the present invention, there is provided a medical appliance comprising a main body having a collar at a base portion of the main body, the collar having a first engagement means; and a protector put on the main body, the protector being shaped like a tubular body having a bottom out of synthetic resin, and the protector having a second engagement means at an opening end thereof
wherein the first engagement means and the second engagement means are engaged to each other before the protector is put off from the main body of the medical appliance.

In a first particular embodiment of the present invention, the first engagement means is a hole made on a collar near a root of the collar, while the second engagement means is a breakable projection having such a thickness that allows insertion of the projection through the hole. A tip portion of the projection has a bulged portion obtained by inserting the projection of the protector put on the main body through the hole and thereafter by heating and deforming the projected tip portion.

In another particular embodiment of the present invention, the first engagement means is a notch formed at the periphery of the collar, while the second engagement means is a L-shaped projection designed to be engaged with the notch and welded or adhered to the notch.

In the first embodiment, when the main body of the medical appliance and the protector are held with fingers and then the protector is twisted, a projection of the protector is easily broken so that at least a bulged portion of the projection is separated or removed from the protector. It is not possible to form a bulged portion again which is so heat-deformed as not to pass through the hole. Accordingly, it can be proved that the protector has not yet been put off from the main body of the medical appliance by the presence of a heat-deformed bulged-portion at a tip of the projection.

In the second embodiment, when the main body of the medical appliance and the protector are held with fingers and then the protector is twisted, the protector is separated from the main body in spite of welding or adhesion between the L-shaped projection and the notch. In that case, when welding or adhesion is too strong to easily separate the protector from the main body, it is preferable to provide a fragible portion between the engaging portion of the protector and the main body of the protector. The frangible portion is easily broken by twisting the protector while holding the appliance with fingers, so that the protector is separated from the main body.

It is not possible to reproduce sufficient engagement between the protector and the main body by using the portion which is so broken as stated above. Accordingly, it can be proved that the protector has not yet been put off from the main body of the medical appliance by ascertaining the presence of welding or adhesion of an engaging portion of the protector put on the main body of the medical appliance.

Now, non-restrictive embodiments of the medical appliance of the present invention are explained based on the drawings attached hereto, wherein
Fig. 1 is a perspective view of a first embodiment of the medical appliance of the present invention,
Fig. 2a is a perspective view of an example of the main body of the medical appliance of the present invention,
Fig. 2b is a perspective view of an example of the protector of the medical appliance of the present invention,
Figs. 3 and 4 are other examples of the main body of the medical appliance of the present invention,
Fig. 5 is a perspective view of a second embodiment of the medical appliance of the present invention;
Fig. 6a is a perspective view of an example of the main body of the second embodiment of the medical appliance of the present invention, and
Fig. 6b is a perspective view of an example of the protector of the second embodiment of the medical appliance of the present invention.

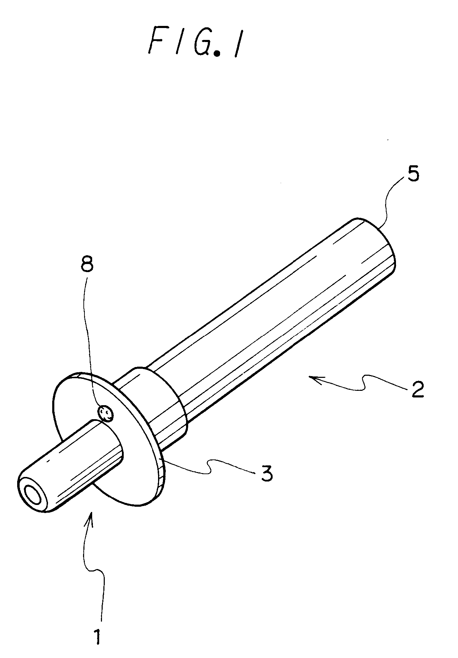

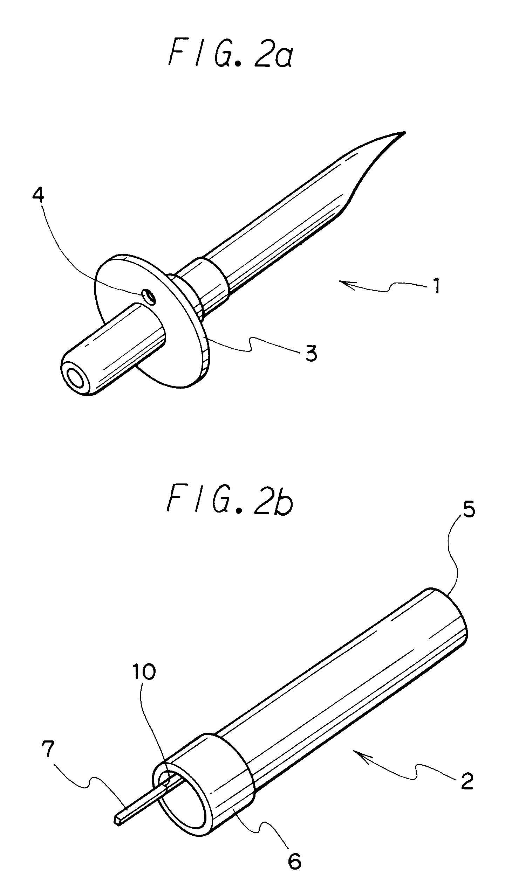

Figs. 1 to 4 show a first embodiment of the present invention. As shown in Figs. 2a and 2b, the medical appliance of the first embodiment of the present invention is composed of a main body 1 of the medical appliance and a protector 2. A bar-like projection 7 (second engagement means) is inserted through a hole 4 (first engagement means) formed in a collar 3 of the main body 1. The collar 3 is formed at a base portion of the main body 1. A part of the projection 7 which projects from the hole 4 is heated and deformed to form a heat-deformed bulged portion 8. The bulged portion 8 is designed not to pass through the hole 4.

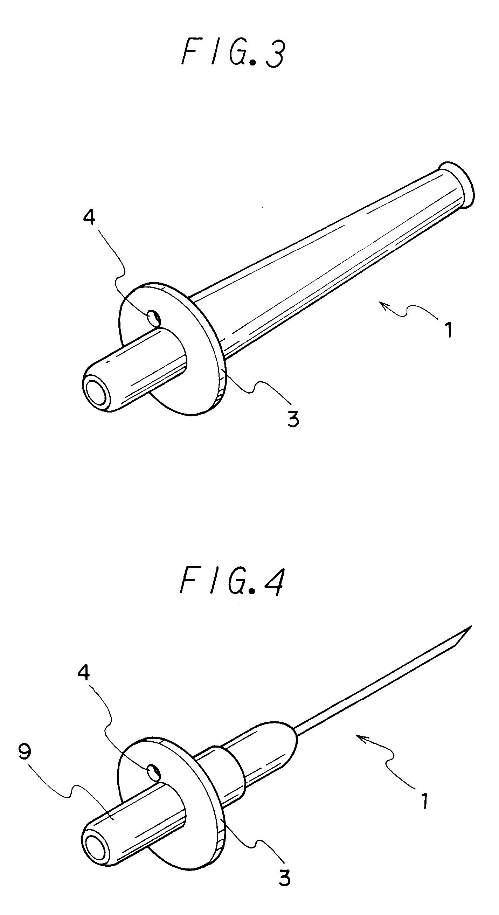

As main body 1 there can be employed a liquid-introducing needle wholly made of synthetic resin as shown in Fig. 2a, a connector made of synthetic resin as shown Fig. 3, and an injection needle having a stainless steel needle as shown in Fig. 4. As mentioned above, the collar 3 is formed at a base portion of the main body 1. That is, when the main body is a needle, the collar is formed at a hub 9. When the main body is a connector, the collar is formed at an end which is opposite to the connecting end of the connector. A hole 4 is formed in the collar 3 near the root of the collar.

The protector 2 comprises a tubular body wholly made of synthetic resin such as polypropylene, polyethlene, polyester, polycarbonate, polystyrene, or acrylonitrile-butadien-styrene copolymer. At an end of the protector 2, a bottom 5 is formed to close the protector 2. The projection 7 is formed at an open end 6 of the protector 2. The projection 7 might be formed at an end surface of the opening of the protector 2, or might be formed at an end surface of an engaging rib formed on the inner wall of the protector 2.

The projection 7 has a thickness that allows the insertion of the projection 7 through the above-mentioned hole 4 formed in the collar 3 of the main body 1. The projection 7 is so formed as to be easily broken. When the projection 7 is made thin, it is possible to cut the projection 7 with a cutter on using the medical appliance. However, it is preferable to form the projection so that it can be easily broken with hands only. By making the projection very thin, there can be obtained a projection which can be easily broken with hands. There can also be obtained a projection which can be easily broken with hands by making the projection from synthetic resin having a relatively low torsional strength such as polypropylene, polystyrene and polyester. Further, a frangible portion such as a nick 10 and a small-diameter portion (not shown) formed at a part of the projection 7 also facilitate the breakage of the projection 7.

The shape of the heat-deformed bulged portion 8 of the projection 7 is preferably globe-shaped or bar-shaped in which the globe or the bar has a larger diameter than the hole 4. Any shape can, however, be employed as long as the projection 7 cannot be pulled out from the hole 4 till a heat-deformed bulged portion of the projection is broken off.

There can be obtained a medical appliance having the same effect as in the present invention by inserting the projection 7 through the hole 4 and then welding a tip portion of the projection 7 to the collar 3. In that case, however, it becomes possible, depending on the manner of breaking, to weld again the tip portion of the projection 7 after the protector is once put off from the main body. Accordingly, the above welding method is not perfect as a method of assuring that a medical appliance is not opened.

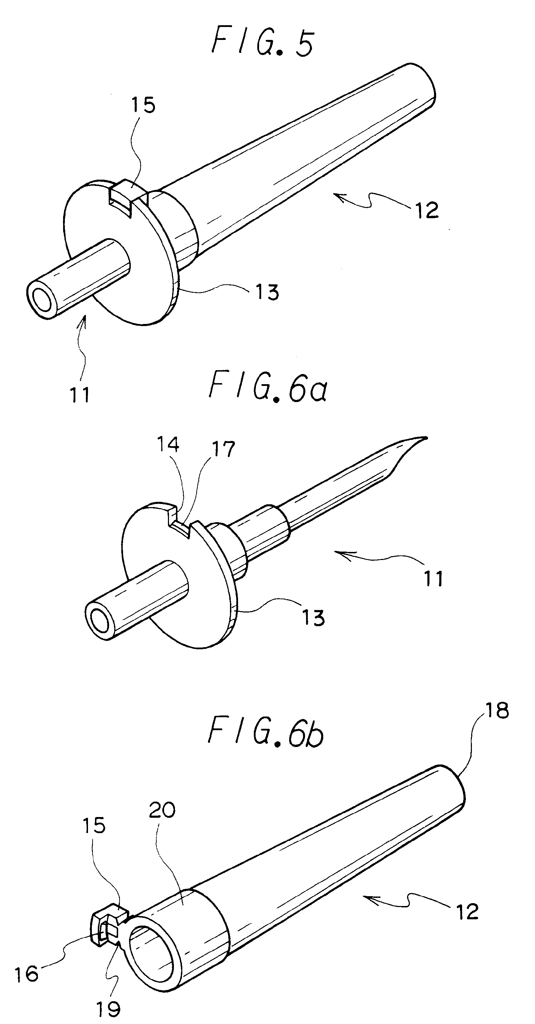

Next, a second embodiment of the present invention is explained based on Figs. 5, 6a and 6b.

As shown in Figs. 6a and 6b, the second embodiment of the present invention is composed of a main body 11 and a protector 12. The medical appliance is arranged by engaging a L-shaped projection 15 (second engagement means) of the protector 12 with a notch 14 (first engagement means) formed at the periphery of a collar 13 of the main body 11 when putting the protector 12 on the main body 11. An engagement portion between a projection 16 of the L-shaped projection 15 and a jaw 17 formed at a radially innermost portion of the notch 14 is then welded or adhered.

When providing a notch 19 for breaking as a fragible portion between an engaging portion of the projection 15 and the main body 20 of the protector 12, the protector 12 can be easily separated from the main body of the medical appliance for using the medical appliance.

As main body 11 there can be employed, for example, a liquid-introducing needle wholly made of synthetic resin as shown in Fig. 6a, a connector made of synthetic resin (not shown), and an injection needle (not shown) having a stainless steel needle. An engaging portion is formed at a base portion of the main body 11. That is, when the main body is a needle, the engaging portion is formed at a hub. When the main body is a connector, the engaging portion is formed at an end which is opposite to the connecting end of the connector.

The protector 12 comprises a tubular body wholly made of synthetic resin such as polypropylene, polyethylene, polyester, polycarbonate, polystyrene, or acrylonitrile-butadien-styrene copolymer. At an end of the protector 12, a bottom 18 is formed to close the protector 12. The projection 16 is formed at one side of the L-shaped projection 15.

As is clear from the above explanation, with a medical appliance of the present invention, it can surely be ascertained that the medical appliance has not yet been opened, in other words, seal of the device has no yet been broken. Accordingly, there can be prevented the danger that an infected medical appliance is used by mistake in the treatement of patients.

## Claims

1. Medical appliance, comprising a main body (1; 11) having a collar (3; 13) at a base portion thereof, the collar having a first engagement means (4; 14), and a protector (2; 12) surrounding a portion of the main body so as to maintain said portion of the main body in sterile condition, the protector having a tubular body with a closed end (5; 18) and being made of synthetic resin, and the protector having a second engagement menas (7; 15) at an open end thereof, wherein the first and second engagement means are engaged to one another in such a manner that the engagement means must be broken before the protector can be removed from the main body.

2. Medical appliance according to claim 1, characterized in that the first engagement means (4) is a hole made in the collar (3) near the root of the collar and the second engagement means (7) is a breakable projection having a thickness that allows insertion of the projection through the hole, the tip of the projection having a bulged portion (8) obtained by inserting the projection of the protector when putting on the main body through the hole and thereafter by heating the projecting tip.

3. Medical appliance according to claim 2, characterized in that at least a part of the projection (7) is frangible.

4. Medical applicance according to claim 1, characterized in that the first engagement means (14) is a notch formed at the periphery of the collar (13) and the second engagement means (15) is a L-shaped projection designed to be engaged with the notch and welded or adhered to the notch.

5. Medical appliance according to claim 4, characterized in that the L-shaped projection (15) has a notch (19) for breaking off the L-shaped projection at its root.

## Patentansprüche

1. Medizinische Einrichtung mit einen Hauptkörper (1; 11), welcher an seinem Unterteil einen Kragen (3; 13) aufweist und dieser Kragen ein erstes Eingriffsmittel (4; 14) enthält, und mit einer Schutzkappe (2; 12), welche einen Teil des Hauptkörpers umgibt, um diesen Teil des Hauptkörpers in einem sterilen Zustand zu erhalten, und in der die Schutzkappe einen schlauchförmigen Körper mit einem geschlossenen Ende (5; 18) aufweist, der aus synthetischem Harz hergestellt ist, und diese Schutzkappe an einem offenen Ende ein zweites Eingriffsmittel (7; 15) aufweist, wobei diese ersten und zweiten Eingriffsmittel so ineinander eingreifen, daß diese Eingriffsmittel abgebrochen werden müssen, bevor die Schutzkappe von dem Hauptkörper abgezogen werden kann.

2. Medizinische Einrichtung nach Anspruch 1,
**dadurch gekennzeichnet, daß**
das erste Eingriffsmittel (4) eine Bohrung ist, welche in den Kragen (3) in der Nähe seiner Wurzel eingebracht ist und daß das zweite Eingriffsmittel (7) ein abbrechbarer Abschnitt ist, der eine Dicke hat, welche es erlaubt, diesen Abschnitt durch diese Bohrung einzuschieben, und daß die Spitze dieses Abschnittes einen ausgebuchteten Teil (8) aufweist, der dadurch erreicht wird, daß der Abschnitt beim Aufsetzen der Schutzkappe auf den Hauptkörper durch die Bohrung geschoben und anschließend die Spitze dieses Abschnittes erwärmt wird.

3. Medizinische Einrichtung nach Anspruch 2,
**dadurch gekennzeichnet, daß**
mindestens ein Teil dieses Abschnittes (7) abbrechbar ist.

4. Medizinische Einrichtung nach Anspruch 1,
**dadurch gekennzeichnet, daß**
das erste Eingriffsmittel (14) eine Kerbe ist, welche an der Peripherie des Kragens (13) ausgebildet ist, und daß das zweite Eingriffsmittel (15) ein L-förmiger Abschnitt ist, welcher mit dieser Kerbe in Eingriff treten kann und an dieser Kerbe verschweißt oder befestigt ist.

5. Medizinische Einrichtung nach Anspruch 4,
**dadurch gekennzeichnet, daß**
der L-förmige Abschnitt (15) eine Kerbe (19) aufweist, um den L-förmigen Abschnitt an seiner Wurzel abzubrechen.

## Revendications

1. Dispositif médical comprenant un corps principal (1; 11) incluant, disposé à sa partie inférieure, un col (3; 13), ledit col comprenant un premier moyen d'engrènement (4; 14), et comprenant un protecteur (2; 12) enfermant une partie dudit corps principal afin de maintenir cette partie en état stérile, ledit protecteur étant composé d'un corps tubulaire avec une extrémité fermée (5; 18) et est réalisé en une résine synthétique, et ledit protecteur comprenant à une extrémité ouverte un second moyen d'engrènement (7; 15), lesdits premier et second moyens d'engrènement étant fixés de telle manière l'un par rapport à l'autre que ces moyens d'engrènement doivent être brisés avant de pouvoir enlever ledit protecteur dudit corps principal.

2. Dispositif médical suivant la revendication 1,
**caractérisé en ce que**
le premier moyen d'engrènement (4) est un trou agencé dans ledit col (3) à proximité de sa racine, et en ce que le second moyen d'engrènement (7) est une partie en saillie brisable d'une épaisseur permettant d'insérer ladite partie en saillie dans ledit trou, et en ce que la pointe de ladite partie en saillie comprend une section bombée (8) obtenue par l'insertion de ladite partie en saillie dudit protecteur, lorsque celui-ci est placé sur ledit corps principal, dans ledit trou et en chauffant ensuite la pointe de ladite partie en saillie.

3. Dispositif médical suivant la revendication 2,
**caractérisé en ce que**
au moins une section de ladite partie en saillie (7) est brisable.

4. Dispositif médical suivant la revendication 1,
**caractérisé en ce que**
le premier moyen d'engrènement (14) est une entaille formée sur la périphérie du col (13), et en ce que le second moyen d'engrènement (15) est une partie en saillie sous forme d'un L qui entre en contact avec ladite entaille et est soudée ou fixée dans cette entaille.

5. Dispositif médical suivant la revendication 4,
**caractérisé en ce que**
ladite partie en saillie sous forme d'un L (15) comprend une entaille (19) permettant de briser ladite partie en saillie à sa racine.
